# FASCICULE DE BREVET EUROPEEN

(11) **EP 2 340 781 B1**
(45) Date de publication et mention de la délivrance du brevet: **20.07.2016**
(21) Numéro de dépôt: 10196084.7
(22) Date de dépôt: 21.12.2010
(51) Int. Cl.: A61B 34/00

(54) **Procédé et appareil de localisation et de visualisation d'une cible par rapport à un point focal d'un système de traitement**
Verfahren und Vorrichtung zur Lokalisation und Visulaisierung eines Ziels in Bezug auf einen Fokuspunkt eines Behandlungssystems
Procedure and device for localistation and visualisation of a target with respect to a focal point of a treatment system

(30) Priorité: 05.01.2010 FR 1050041
(43) Date de publication de la demande: 06.07.2011
(73) Titulaire: EDAP TMS France, 69120 Vaulx-en-Velin (FR)
(72) Inventeur: Peyrard, André, 42320, SAINT CHRISTOPHE EN JARES (FR); Vurpillot, Claire, 69100, VILLEURBANNE (FR); Chatre, René, 69200, VENISSIEUX (FR)
(74) Mandataire: Thibault, Jean-Marc

(56) Documents cités:
- DE-A1-102004 006 021
- DE-A1-102006 060 070
- US-A- 5 944 663

## Description

L'objet de l'invention concerne le domaine technique de la visualisation et de la localisation d'une structure anatomique ou d'une concrétion dans le corps d'un mammifère et en particulier un être humain.

La localisation et la visualisation des structures anatomiques ou des concrétions appelées d'une manière générale des cibles dans la suite de la description, sont couramment réalisés par des dispositifs d'imagerie reposant sur différents procédés telles les méthodes échographiques utilisant les ultrasons, ou les méthodes tomographiques basées sur les rayons X ou sur la résonnance magnétique (IRM). Ces dispositifs d'imagerie permettent ainsi de positionner un système de traitement thérapeutique par rapport à la cible afin de la traiter par la focalisation, selon un point focal, d'ondes de chocs ou ultrasonores.

Dans l'état de la technique, il est connu par le document DE 10 2006 060070, un appareil de traitement comportant un système de traitement par ondes de pression acoustiques, à l'aide d'un système d'imagerie de type à rayons X, même si un tel document prévoit la possibilité d'un système d'imagerie de type acoustique. Un tel système d'imagerie rayons X est monté sur une structure porteuse mobile par rapport au bâti de l'appareil. Un tel appareil comporte également un système de repérage à distance permettant de détecter la position du système de traitement et du système d'imagerie. L'appareil comporte un écran d'affichage sur lequel sont visualisés le point focal du système de traitement et la cible à traiter. Le système de traitement est déplacé pour mettre en coïncidence le point focal et la cible à traiter.

Dans le même sens, le document DE 10 2004 006021 décrit de manière analogue, un appareil de traitement dans lequel aucune liaison mécanique n'existe entre le système d'imagerie, le système de thérapie et la table de support du patient. Ce document décrit une technique permettant d'aligner, à l'aide du système d'imagerie à rayons X, le foyer du système de thérapie sur la cible à traiter. Le document enseigne de prendre une série de clichés RX réalisés selon deux incidences et à repérer sur chacun des clichés, l'iso-centre du système d'imagerie, le point focal du système de thérapie et la cible, puis à mettre en correspondance le point focal et la cible par le mouvement de l'un ou de l'autre sachant que tous les degrés de libertés associés à ces mouvements sont munis de codeurs permettant de connaître les valeurs de déplacement.

Il est à noter que le principe physique de projection d'images d'un système d'imagerie du type à rayons X permet le suivi visuel de l'alignement de la cible et du point focal puisque ce système d'imagerie est en permanence orienté pour suivre le volume situé autour du point focal. Toutefois, d'une manière générale, les dispositifs d'imagerie de type rayons X sont coûteux, complexes et encombrants de sorte qu'il apparaît difficile de les utiliser en combinaison avec les systèmes de traitement. Par ailleurs, le suivi par rayons X de l'alignement de la cible et du point focal nécessite une irradiation continue du patient, néfaste pour ce dernier et le personnel soignant.

A l'inverse, les systèmes d'échographie sont plus compacts, peu coûteux et peuvent facilement être utilisés en association avec les systèmes de traitement thérapeutiques. Par ailleurs, les systèmes d'échographie n'émettent pas de radiation ionisantes.

Dans l'état de la technique, il existe de nombreuses réalisations d'appareils combinant une sonde ultrasonore d'un système d'imagerie et un système de traitement par focalisation d'ondes sur un point focal. D'une manière générale, le dispositif de localisation permet de visualiser sur l'image, la cible à traiter et le point focal de l'appareil de traitement. La mise en correspondance de la cible et du point focal est obtenue en déplaçant soit le point focal du système de traitement, soit la cible c'est-à-dire la table où repose le patient. Cette mise en correspondance nécessite bien entendu la connaissance de l'orientation du plan image par rapport aux axes de déplacement mécanique de la table ou de l'appareil de traitement. Par la mesure sur l'image des coordonnées de la cible et du point focal et de la connaissance de l'orientation du plan image, il est possible de calculer la distance séparant la cible et le point focal et de déplacer le point focal à savoir le dispositif de traitement ou la cible afin de les mettre en coïncidence.

Dans l'état de la technique, il est connu ainsi, notamment par les brevets EP 0 404 871 ou US 5 078 124 de positionner la sonde ultrasonore du système d'imagerie de telle sorte qu'elle vise en permanence le point de focalisation de l'appareil de traitement afin que ce point de focalisation apparaisse de manière continue sur l'image. L'axe du plan image passe par le point de focalisation de l'appareil de thérapie. Pour faire apparaître la cible sur le même plan image, l'opérateur peut soit déplacer le patient de manière que l'image de la cible apparaisse sur l'image échographique, soit déplacer la sonde échographique autour de son axe visant le point de focalisation jusqu'à faire apparaître la cible sur l'image échographique.

Il doit être considéré que la sonde échographique est toujours solidaire d'un support mécanique visant le point de focalisation de l'appareil de traitement. Cette contrainte est extrêmement pénalisante pour l'opérateur dans la mesure où il apparaît difficile dans certains cas de trouver la cible compte tenu de la limitation des mouvements de la sonde et/ou du patient afin d'obtenir sur une même image la cible et le point de focalisation. Par ailleurs, il apparaît difficile d'ajuster le plan de coupe image échographique par rapport à l'anatomie de la cible observée afin d'obtenir la meilleure définition possible des contours ou de l'intérieur de la cible.

Il est à noter que le brevet US 5 944 663 décrit un procédé de traitement comportant une source ultrasonore focalisée qui n'est pas associée à un dispositif d'imagerie. En effet, l'imagerie du patient est réalisée dans une phase préalable à la phase de traitement. Ce procédé prévoit de mettre en correspondance le repère du dispositif d'imagerie avec le repère du dispositif de thérapie. Cette mise en correspondance est réalisée par la présence de marqueurs positionnés sur la table sur laquelle le patient est allongé et qui seront visualisés par le système de repérage dans chacune des phases d'imagerie puis de thérapie. Les images du patient sont positionnées par rapport aux marqueurs de la table qui sont repérés dans un repère associé au dispositif d'imagerie. Ces mêmes marqueurs sont ensuite positionnés dans le repère associé au dispositif de thérapie. La sonde de thérapie est positionnée dans ce même repère grâce à un second jeu de marqueurs solidaire du dispositif de thérapie. Ainsi, un tel procédé permet de disposer, dans un même repère, de l'ensemble des informations permettant de mettre en correspondance, l'imagerie réalisée sur le patient et la zone de traitement pour afficher dans le volume image, la position du point focal.

L'inconvénient principal de cette technique concerne le fait que l'imagerie n'est pas réalisée en temps réel puisque l'imagerie est réalisée dans une phase préalable à la phase de traitement. Cette technique ne permet pas de suivre et de confirmer le positionnement correct du dispositif de thérapie par rapport à la zone de visée. Cette pratique repose sur l'immobilité totale du patient pendant la phase de traitement, ce qui apparaît difficile en pratique à mettre en oeuvre.

La présente invention vise donc à remédier aux inconvénients énoncés ci-dessus en proposant une nouvelle technique offrant l'avantage de faciliter l'opération de localisation d'une cible anatomique à l'aide d'un système d'imagerie ultrasonore tout en permettant de positionner avec précision, un système de traitement par ondes de pression acoustiques pour traiter ladite cible.

Pour atteindre un tel objectif, l'objet de l'invention concerne un procédé de localisation et de visualisation d'une cible appartenant à un mammifère, en particulier à un être humain par rapport à un point focal d'un système de traitement par ondes de pression acoustiques, à l'aide d'un système d'imagerie ultrasonore comportant une sonde ultrasonore et au moins un écran d'affichage d'images, la cible et/ou le système de traitement étant déplacés par des moyens de déplacement pour mettre en coïncidence la cible et le point focal. L'invention est décrite dans le jeu de revendications.

Selon l'invention le procédé comporte les étapes suivantes :
- réaliser une sonde ultrasonore mobile dans l'espace indépendante mécaniquement du système de traitement et localisée par un système de repérage à distance lorsqu'elle se trouve située dans son volume de détection,
- déplacer la sonde ultrasonore dans le volume de détection pour visualiser la cible dans une image affichée sur l'écran,
- assurer l'enregistrement simultanément, d'une part d'une image ultrasonore dans laquelle apparaît l'image de la cible, et d'autre part, de la position de la sonde ultrasonore,
- sélectionner sur l'image ultrasonore enregistrée, la position de l'image de la cible afin de déterminer la position virtuelle de la cible,
- déterminer simultanément d'une part, la position du point focal du système de traitement à l'aide du système de repérage à distance et d'autre part la position des moyens de déplacement de la cible et/ou du système de traitement,
- calculer, à partir de la position virtuelle de la cible et des positions du point focal et des moyens de déplacement, les valeurs de déplacement pour les moyens de déplacement pour permettre une mise en coïncidence de la position virtuelle de la cible avec le point focal,
- et déplacer la cible et/ou le point focal à l'aide des moyens de déplacement et selon les valeurs de déplacement calculées, de manière à mettre en coïncidence la position virtuelle de la cible avec le point focal.

Le procédé selon l'invention comporte également en combinaison l'une et/ou l'autre des caractéristiques suivantes :
- déterminer la position des moyens de déplacement à l'aide du système de positionnement à distance,
- assurer l'affichage des valeurs de déplacement calculées pour les moyens de déplacement et à mettre à jour ces valeurs de déplacement affichées en fonction du déplacement réalisé,
- visualiser sur l'écran, l'image virtuelle de la cible avec l'image du point focal, et leur déplacement lors de la mise en coïncidence de l'image virtuelle de la cible avec le point focal par les moyens de déplacement,
- assurer l'affichage de deux images virtuelles réalisées dans deux plans sécants et faisant apparaître dans chacune d'elles, l'image virtuelle de la position de la cible et l'image virtuelle de la position du point focal, ainsi que leurs déplacements relatifs consécutifs au mouvement des moyens de déplacement,
- à l'aide du système de positionnement à distance, à visualiser l'orientation du volume focal du système de traitement,
- une étape de validation de la coïncidence du point focal et de la cible, consistant :
   - à déplacer la sonde ultrasonore pour localiser la cible pour permettre d'afficher sur l'écran, l'image de la cible,
   - à assurer l'enregistrement de la position du point focal et l'affichage de l'image du point focal pour vérifier la coïncidence entre le point focal et la cible en vue de commander le système de traitement,
- l'étape de validation consiste, en cas de décalage entre le point focal et la cible :
   - à déplacer la sonde ultrasonore pour visualiser la cible dans une image affichée sur l'écran,
   - à enregistrer simultanément, d'une part l'image dans laquelle apparaît l'image de la cible et d'autre part, la position de la sonde ultrasonore,
   - à sélectionner sur l'image enregistrée, la position de l'image de la cible,
   - à déterminer simultanément d'une part, la position du point focal et d'autre part, la position des moyens de déplacement,
   - à calculer les valeurs de déplacement pour les moyens de déplacement pour permettre une mise en coïncidence du point focal avec la position virtuelle de la cible,
   - et à déplacer la cible et/ou le point focal à l'aide des moyens de déplacement de manière à mettre en coïncidence la position virtuelle de la cible avec le point focal,
- à afficher l'image du point focal dans une position indépendante de l'axe de visée de la sonde ultrasonore.

Un autre objet de l'invention concerne un appareil de localisation et de visualisation d'une cible appartenant à un mammifère, en particulier à un être humain, cet appareil comportant :
- un système de traitement par ondes de pression acoustiques dirigées vers un point focal,
- des moyens de déplacement de la cible et/ou du système de traitement, pour mettre en coïncidence la cible et le point focal,
- des moyens pour déterminer la position de ces moyens de déplacement,
- un système d'imagerie ultrasonore comportant une sonde ultrasonore et des moyens de formation d'images permettant de visualiser sur au moins un écran, la cible et le point focal.

Selon l'invention, l'appareil comporte :
- une sonde ultrasonore mobile dans l'espace et indépendante mécaniquement du système de traitement,
- un système de repérage d'objets à distance permettant de localiser le point focal du système de traitement et la sonde ultrasonore lorsque la sonde ultrasonore et le système de traitement sont situés dans le volume de détection dudit système de repérage,
- une unité de calcul et de commande comportant :
   - des moyens pour enregistrer simultanément, d'une part, une image ultrasonore dans laquelle apparaît l'image de la cible et d'autre part, la position de la sonde ultrasonore détectée et calculée par le système de repérage à distance,
   - des moyens pour sélectionner sur l'image ultrasonore enregistrée, la position de l'image de la cible afin de déterminer la position virtuelle de la cible,
   - des moyens pour déterminer simultanément, d'une part, la position du point focal à partir du système de repérage à distance et d'autre part, la position des moyens de déplacement,
   - des moyens pour calculer, à partir de la position virtuelle de la cible et des positions du point focal et des moyens de déplacement, les valeurs de déplacement pour les moyens de déplacement pour permettre une mise en coïncidence de la position virtuelle de la cible avec le point focal,
   - et des moyens pour commander, selon les valeurs de déplacement calculées, les moyens de déplacement pour mettre en coïncidence la position virtuelle de la cible avec le point focal.

L'appareil selon l'invention comporte également en combinaison l'une et/ou l'autre des caractéristiques suivantes :
- les moyens pour déterminer la position des moyens de déplacement font partie du système de repérage à distance,
- le système de repérage à distance comporte au moins deux caméras de préférence infrarouge comportant un volume de détection dans lequel sont placés un premier, un deuxième et voire un troisième marqueurs montés solidaires respectivement de la sonde ultrasonore, du système de traitement, et des moyens de déplacement de la cible,
- le troisième marqueur monté solidaire des moyens de déplacement est orienté selon les axes de déplacement desdits moyens de déplacement,
- le système de repérage à distance comporte un marqueur de calibration pour le point focal,
- des moyens assurant l'affichage des valeurs de déplacement calculés pour les moyens de déplacement, ces valeurs affichées étant mises à jour en fonction du déplacement réalisé,
- des moyens pour visualiser sur l'écran l'image virtuelle de la cible, l'image du point focal et leur déplacement lors de la mise en coïncidence de l'image virtuelle de la cible avec le point focal, par les moyens de déplacement,
- des moyens pour afficher deux images virtuelles réalisées dans deux plans sécants et faisant apparaître dans chacune d'elles, l'image virtuelle de la position de la cible et l'image virtuelle de la position du point focal, ainsi que leurs déplacements relatifs consécutifs au mouvement des moyens de déplacement,
- les moyens d'affichage forment une image affichant le plan image délivré par la sonde ultrasonore et le point focal.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en perspective illustrant un exemple de réalisation d'un appareil de localisation et de visualisation conforme à l'invention.
La **Figure 2** est une vue schématique illustrant des détails caractéristiques de l'appareil de localisation et de visualisation conforme à l'invention.
Les **Figures 3A** et **3B** sont des sont des vues schématiques montrant une image ultrasonore dans laquelle apparaît respectivement l'image de la cible et les images de la cible et du point focal.
Les **Figures 3C** et **3D** sont des vues schématiques d'images virtuelles dans chacune desquelles apparaissent l'image de la cible et l'image du point focal.
Les **Figures 3E** et **3F** sont des vues schématiques d'images virtuelles dans chacune desquelles apparaissent en coïncidence, l'image de la cible et l'image du point focal.
Les **Figures 4A** et **4B** sont des vues schématiques d'images virtuelles respectivement conforme à l'invention et de l'art antérieur.
Les **Figures 5A** et **5B** sont des vues schématiques d'images virtuelles montrant l'orientation du plan image.

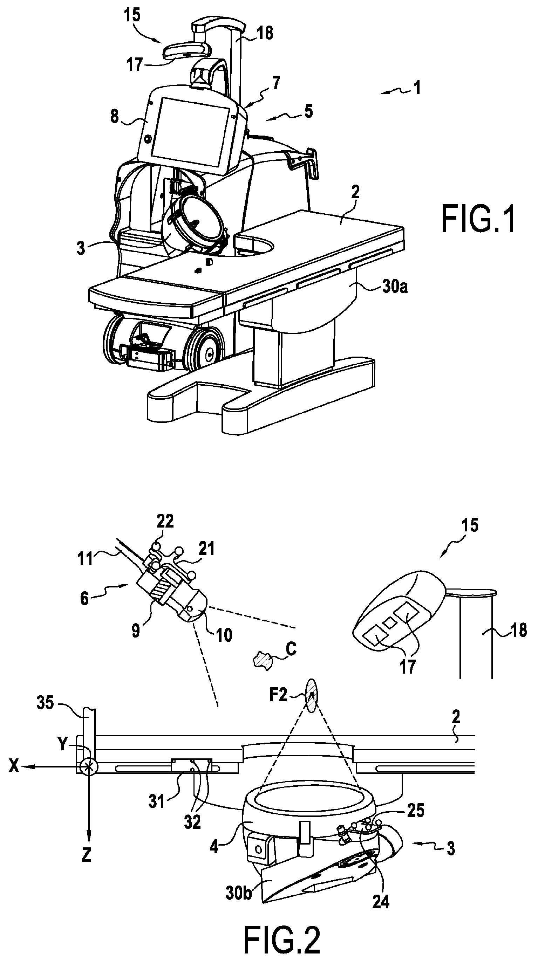

Les **Fig. 1** et **2** illustrent un appareil **1** de localisation et de visualisation d'une cible **C** telle une structure anatomique ou une concrétion appartenant à un mammifère et en particulier à un être humain. De manière classique, l'appareil **1** comporte une table de support **2** d'un patient portant la cible **C.** La cible **C** est destinée à être traitée par l'intermédiaire d'un système de traitement **3** par ondes de pression acoustiques dirigées vers un point focal **F2.** Un tel système de traitement **3** assure la focalisation d'ondes de chocs ou d'ondes ultrasonores. De manière classique, ce système de traitement **3** comporte un générateur **4** conçu pour focaliser des ondes ultrasonores ou des ondes de chocs sur un point focal **F2.** Le système de traitement **3** n'est pas décrit plus précisément car il est bien connu de l'homme du métier et ne fait pas partie précisément de l'objet de l'invention.

L'appareil **1** conforme à l'invention comporte également un système d'imagerie ultrasonore **5** comportant une sonde ultrasonore ou d'échographie **6,** reliée à des moyens de formation d'images **7** sur un écran **8.** Un tel système d'imagerie ultrasonore **5** permet l'affichage sur l'écran **8** d'au moins une image ultrasonore ou échographique obtenue par la sonde **6**. Selon une variante préférée de réalisation, l'écran **8** est du type tactile. Ce système d'imagerie ultrasonore **5** n'est pas décrit plus précisément car un tel système est bien connu de l'homme du métier des ultrasons ou de l'échographie et ne sera pas décrit plus en détail.

Le système d'imagerie ultrasonore **5** est relié à une unité de calcul et de commande faisant partie de l'appareil **1** conforme à l'invention. Cette unité de calcul et de commande comporte différents moyens notamment d'enregistrement, de calcul, d'affichage, de commande adaptés pour effectuer diverses fonctions qui apparaitront plus précisément dans la suite de la description. De même, l'appareil **1** est relié au système de traitement **3** pour piloter son fonctionnement.

Selon une caractéristique de l'invention, la sonde ultrasonore **6** est indépendante mécaniquement par rapport au système de traitement **3.** Tel que cela ressort plus précisément de la **Fig. 2****,** cette sonde ultrasonore **6** comporte un corps principal **9** pourvu à sa partie distale, d'un transducteur ultrasonore **10.** Cette sonde ultrasonore **6** est recouverte d'une coque formant une poignée de préhension. La sonde ultrasonore **6** est équipée à sa partie proximale d'un câble de connexion électrique **11** avec les moyens de formation d'images **7.** Cette sonde ultrasonore **6** est donc reliée uniquement par le câble de connexion électrique **11** aux moyens de formation d'images **7.** La sonde ultrasonore **6** n'est donc pas liée mécaniquement au système de traitement **3.** La sonde ultrasonore est donc libre mécaniquement par rapport au système de traitement **3.**

Il doit être compris que la sonde ultrasonore **6** est libre de déplacement dans l'espace offrant ainsi à l'opérateur une grande liberté de mouvements dans le déplacement de la sonde ultrasonore lui permettant d'ajuster en permanence le plan de coupe image par rapport à l'anatomie du patient afin d'obtenir la meilleure définition possible d'un organe ou de la cible **C** à l'intérieur d'un organe.

L'appareil **1** selon l'invention comporte également un système de repérage d'objets à distance **15** permettant de localiser en particulier le point focal **F2** du système de traitement **3** et la sonde ultrasonore **6** lorsque la sonde ultrasonore **6** et le point focal **F2** sont situés dans un volume de détection du système de repérage **15.** Le système de repérage à distance **15** peut faire appel à tous types de techniques de détection. Avantageusement, le système de repérage **15** est du type à détection d'un rayonnement électromagnétique infrarouge.

Selon la variante avantageuse de réalisation, le système de repérage **15** comporte au moins deux caméras **17** à détection infrarouge montées sur un support **18.** Les caméras **17** sont orientées de manière à définir un volume de détection dans lequel est placé un premier marqueur **21** fixé sur la sonde ultrasonore **6**. De préférence, le premier marqueur **21** est fixé sur le corps **9** de la sonde ultrasonore **6** afin de ne pas gêner la préhension de la sonde ultrasonore. De même, la préhension de la sonde ultrasonore **6** ne doit pas masquer le premier marqueur **21** au regard du système de repérage **15.**

Le premier marqueur **21** comporte au moins trois et dans l'exemple illustré quatre sphères réfléchissantes **22** dont les positions relatives entre elles sont connues. La quatrième sphère réfléchissante **22** est redondante avec les trois autres afin d'améliorer la précision du repérage spatial. Ce premier marqueur **21** est visualisé simultanément par les deux caméras **17** permettant de l'observer selon deux incidences différentes. Le système de repérage **15** dispose ainsi de deux images contenant chacune des sphères réfléchissantes du premier marqueur **21.** L'analyse de ces images permet d'extraire les six informations de position qui sont nécessaires au calcul des six degrés de liberté de position et d'orientation de la sonde ultrasonore **6** à savoir trois degrés de liberté de position et trois degrés d'orientation.

Un tel système de repérage **15** permet de connaître la position de la sonde ultrasonore **6** dans l'espace et en particulier dans le volume de détection des caméras à infrarouge **17.** La position et l'orientation de la sonde ultrasonore **6** sont calculées dans un repère associé par exemple aux caméras **17.** La position de la sonde ultrasonore **6** peut ainsi être représentée par un vecteur auquel sont associées une position et une direction.

Selon une autre caractéristique de l'invention, le système de repérage d'objets à distance **15** permet également de localiser le point focal **F2** du système de traitement **3** lorsque le point focal est situé dans le volume de détection des caméras **17.** Le système de traitement **3** est ainsi équipé d'un deuxième marqueur **24** comportant au moins trois et dans l'exemple illustré quatre sphères réfléchissantes **25** dont les positions relatives entre elles sont connues. Ce deuxième marqueur **24** est également visualisé simultanément par les deux caméras **17** permettant de l'observer selon deux incidences différentes. Le système de repérage **15** est apte à calculer la position spatiale du système de traitement **3** dans le même repère que celui associé au premier marqueur **21** fixé sur la sonde ultrasonore **6.**

Dans l'exemple illustré, le deuxième marqueur **24** est fixé sur le corps du générateur **4** et représente donc indirectement la position du point focal **F2.** Il apparaît ainsi nécessaire d'établir la matrice de transfert donnant la position du point focal **F2** par rapport au deuxième marqueur **24.** L'établissement de cette matrice de transfert est réalisé lors d'une phase de calibration mise en oeuvre lors de la fabrication ou lors de l'installation de l'appareil **1.** Cette phase de calibration consiste à pointer le point focal **F2** avec un outil de calibration non représenté mais équipé d'un autre marqueur du type des premier et deuxième marqueurs **21**, **24** et à calculer la matrice de transfert entre ce marqueur et le deuxième marqueur **24.**

Il est à noter que seule la position du point focal **F2** dans le volume de détection est nécessaire à la mise en correspondance du point focal **F2** et de la cible **C.** Selon une variante avantageuse de réalisation, le système de repérage **15** permet d'accéder à l'orientation spatiale du deuxième marqueur **24.** Aussi, cette information d'orientation spatiale est utilisée pour représenter ou visualiser la direction du cône acoustique et l'orientation du volume focal ou de la propagation des ondes acoustiques et de l'afficher sur un écran, par exemple l'écran **8.**

Le système de repérage **15** fournit, à l'appareil **1,** les informations de localisation de la sonde ultrasonore **6** et du point focal **F2.** L'appareil **1** comporte des moyens d'enregistrement de la position de la sonde ultrasonore **6** et de la position du point focal **F2.** L'appareil **1** comporte également des moyens pour assurer l'affichage sur l'écran **8,** de l'image de la position de la cible **C** et de l'image de la position du point focal **F2** comme cela sera expliqué dans la suite de la description.

L'appareil **1** selon l'invention comporte également des moyens de déplacement de la cible **C** et/ou du système de traitement **3** pour mettre en coïncidence la cible **C** et le point focal **F2.** Les moyens de déplacement sont du type manuel ou motorisé. En ce qui concerne la table de support **2,** les organes de déplacement **30a** assurent le mouvement de la table selon trois directions perpendiculaires **X, Y, Z** correspondant à un déplacement respectivement dans le sens longitudinal de la table, le sens transversal de la table et dans le sens vertical. Dans la suite de la description, il est considéré que les moyens de déplacement **2-30a** permettent le déplacement de la cible **C,** mais l'objet de l'invention s'applique de manière identique aux moyens de déplacement **30b** du système de traitement **3.**

L'appareil **1** selon l'invention comporte également des moyens permettant de déterminer la position des moyens de déplacement **2-30a;30b** de la cible **C** et/ou du point focal **F2.** Il est à noter que la position du point focal **F2** est détectée par le système de repérage **15** de sorte que son déplacement est suivi par le deuxième marqueur **24.** Le déplacement de la cible **C** est assuré par le déplacement de la table de support **2** du patient de sorte que le déplacement de la cible **C** peut être suivi par la détection du déplacement de la table **2.**

Une première solution est d'équiper la table **2** de capteurs ou de codeurs de déplacement en vue de déterminer la position de la cible **C** à l'aide d'une matrice de transfert, dans le repère du point focal **F2.** Pour déterminer cette matrice de transfert, il est nécessaire de connaître l'orientation des axes de déplacement de la table **2** dans le repère du système de repérage à distance **15.** La table **2** et le système de repérage à distance **15** et notamment ses caméras **17** doivent alors occuper des positions relatives invariables dès lors que la matrice de transfert est établie. Il faut alors avoir recours à un support **18** rigide et procéder très régulièrement à des contrôles et à des recalibrations éventuelles.

Pour éviter ces contrôles, l'objet de l'invention prévoit selon une caractéristique avantageuse de réalisation, que les moyens pour déterminer la position des moyens de déplacement **2-30a;30b** font partie du système de repérage à distance **15.** Selon cette variante avantageuse de réalisation, la table **2** est équipée d'un troisième marqueur **31** visible également par les deux caméras **17.** Ce troisième marqueur **31** comporte au moins trois et dans l'exemple illustré, quatre sphères réfléchissantes **32** dont les positions relatives entre elles sont connues. Le système de repérage **15** détermine ainsi la position spatiale de la table **2** dans le même repère que celui associé aux premier **21,** et deuxième **24** marqueurs. La détermination de la position de la table **2** et par suite du mouvement de la cible **C** permet de suivre le déplacement de la table **2** jusqu'à la mise en correspondance de la cible **C** et du point focal **F2.**

Avantageusement, ce troisième marqueur **31** est orienté selon les trois axes de déplacement de la table à savoir les axes **OX, OY, OZ** permettant de calculer le déplacement à réaliser dans le repère **X, Y, Z** de support du patient, sans avoir à calibrer l'orientation des déplacements de la table **2** par rapport à l'orientation du troisième marqueur **31.**

Il est à noter que cette solution offre l'avantage que la position des caméras **17** peut être quelconque et variable d'une procédure de localisation à une autre.

Le système de repérage **15** transfère à l'appareil **1** les informations relatives à la position des moyens de déplacement **2**-**30a, 30b.** L'appareil **1** est apte à enregistrer la position des moyens de déplacement et à afficher sur l'écran **8,** la trajectoire des moyens de déplacement à savoir la trajectoire du point focal **F2** ou la trajectoire de la cible **C,** c'est-à-dire leur position à chaque instant pendant leur déplacement.

L'appareil **1** décrit ci-dessus permet de mettre en oeuvre une méthode nouvelle et originale de localisation et de visualisation de la cible **C.**

Un tel procédé consiste à installer le patient sur la table **2** et à le positionner grossièrement par rapport au système de traitement **3.** Après l'installation du patient, le système de repérage **15** est activé et détecte en permanence la position des premier **21,** deuxième **24** et troisième **31** marqueurs. Le système de repérage **15** calcule ainsi en permanence la position de ces trois marqueurs dans le repère associé par exemple aux caméras **17,** de sorte que les informations sur les positions de la sonde ultrasonore **6,** du point focal **F2** et de la table **2** sont à chaque instant disponibles.

Après avoir pris en main la sonde ultrasonore **6,** l'opérateur recherche la cible **C** dans le patient à l'aide de l'affichage de l'image ultrasonore apparaissant sur l'écran **8.** Cette phase de recherche est facilitée compte tenu que la sonde ultrasonore **6** est indépendante mécaniquement du système de traitement **3.** L'opérateur dispose ainsi d'une grande liberté de mouvements dans le déplacement de la sonde ultrasonore **6** lui permettant d'ajuster en permanence le plan de coupe image par rapport à l'anatomie de l'organe observé. Bien entendu, le patient et les caméras **17** sont placés de manière que lorsque la sonde ultrasonore **6** localise la cible **C,** la sonde ultrasonore **6** se trouve placée dans le volume de détection du système de repérage **15.**

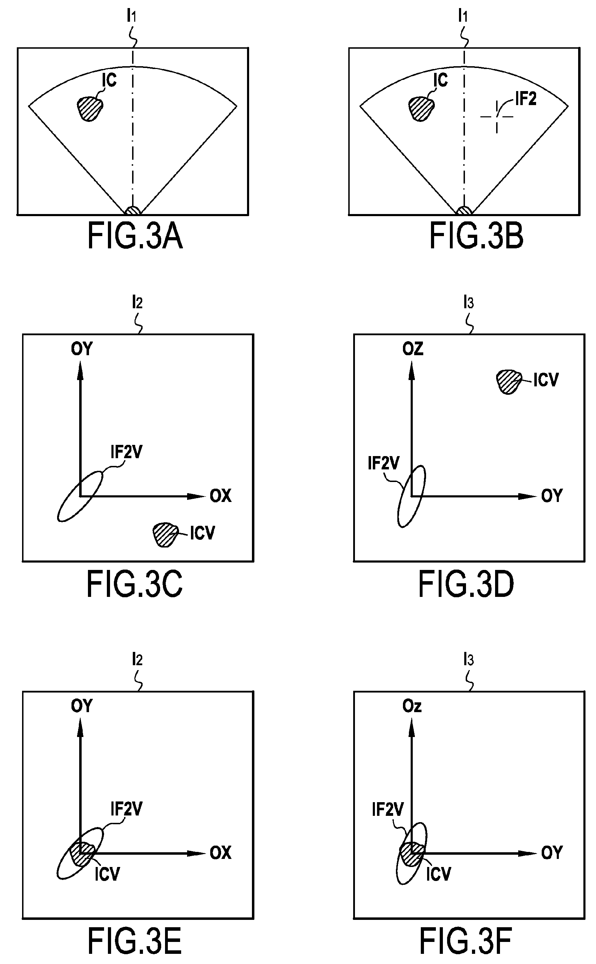

Le système d'imagerie ultrasonore **5** affiche sur l'écran **8,** pendant toute la recherche de la cible **C,** l'image échographique obtenue par la sonde ultrasonore **6.** Tel que cela ressort de la **Fig. 3A****,** l'écran **8** affiche ainsi l'image ultrasonore **I₁** dans laquelle apparaît l'image **IC** de la cible **C.** L'opérateur choisit l'image **I₁** dans laquelle lui apparaît, de manière la plus distinctive, l'image **IC** de la cible **C.** Cette image **I₁** est sélectionnée et enregistrée par l'opérateur par tous moyens appropriés comme par exemple par l'intermédiaire d'un organe de commande telle qu'une pédale. Simultanément, à l'enregistrement de l'image **I₁** choisie par l'opérateur, la position de la sonde ultrasonore **6** détectée et calculée par le système de repérage **15** est enregistrée. Cet enregistrement est réalisé lors de l'activation de l'organe de commande. L'image sélectionnée **I₁** est gelée, c'est-à-dire, qu'elle reste affichée sur l'écran **8.**

La sonde ultrasonore **6** est alors reposée librement sans contrainte de positionnement par rapport au système de repérage **15.**

Tel que cela ressort plus précisément de la **Fig. 3B****,** il est à noter que l'image **IF2** du point focal **F2** peut apparaître dans l'image ultrasonore **I₁** lorsque le point focal est situé dans le plan de l'image ultrasonore. Ainsi, selon une caractéristique de l'objet de l'invention, l'affichage de l'image **IF2** du point focal **F2** est réalisé uniquement lorsque le plan image est sécant au point focal **F2.** L'image **IF2** du plan focal **F2** apparaît ainsi de manière fugitive sur l'écran **8** à la différence de l'art antérieur où l'image ultrasonore affiche constamment l'image du point focal **F2** puisque le plan de l'image ultrasonore est toujours sécant au point focal **F2** en raison de la liaison mécanique entre la sonde ultrasonore et le dispositif de traitement.

L'étape suivante du procédé selon l'invention consiste à sélectionner sur l'image enregistrée **I₁,** la position de l'image **IC** de la cible **C** de manière que l'appareil **1** puisse déterminer la position de la cible **C** à partir de la position de la sonde ultrasonore **6** enregistrée simultanément à l'enregistrement de l'image **I₁**. Dans la mesure où la position de la cible **C** est déterminée à partir d'une image enregistrée, il est considéré dans la suite de la description que cette position correspond à une position virtuelle de la cible **C.** La sélection de la position de l'image **IC** de la cible est avantageusement réalisée en pointant l'image **IC** de la cible directement sur l'écran tactile **8.** Bien entendu, cette sélection peut être réalisée différemment avec l'aide par exemple d'une souris pointant l'image **IC** de la cible.

Simultanément ou postérieurement à la sélection de la position de l'image **IC** de la cible sur l'image enregistrée **I₁**, la position du point focal **F2** déterminée par le système de repérage à distance **15** est enregistrée simultanément à l'enregistrement de la position des moyens de déplacement **2-30a, 30b** de la cible **C** et/ou du système de traitement **3.** Dans l'exemple considéré, sont enregistrées simultanément :
- la position du deuxième marqueur **24** donnant la position du point focal **F2,**
- la position du troisième marqueur **31** donnant la position de la table **2** de déplacement de la cible **C.**

La position virtuelle de la cible **C,** ainsi que les positions du point focal **F2** et de la table **2** sont connus dans un même repère. L'appareil **1** réalise des calculs à partir des informations sur la position virtuelle de la cible **C** et sur les positions du point focal **F2** et de la table **2,** pour déterminer les valeurs de déplacement des moyens de déplacement, pour amener la cible **C** et le point focal **F2** en correspondance. Selon une variante préférée de réalisation, seule la table **2** est déplacée pour permettre d'amener en correspondance la cible **C** et le point focal **F2.**

La cible **C** et/ou le point focal **F2** est déplacé pour assurer cette mise en coïncidence. Dans l'exemple illustré, c'est la table **2** qui est déplacée. Lorsque la table **2** est motorisée de manière automatique, l'opérateur déclenche l'étape de mise en coïncidence de sorte que l'appareil **1** pilote la table **2** selon les valeurs de déplacement calculées pour amener en correspondance la cible **C** et le point focal **F2.**

Dans le cas où la mise en correspondance est réalisée manuellement par l'opérateur, ce dernier est aidé par l'affichage des valeurs de déplacement calculées. Ainsi, il peut être prévu d'afficher sur l'écran **8** les valeurs de déplacement de la table **2** et de mettre à jour continuellement ces valeurs en fonction du déplacement de la table **2.** L'opérateur déplace ainsi la table **2** jusqu'à ce que les valeurs calculées deviennent nulles.

De manière avantageuse, il peut être prévu, en combinaison ou non de l'affichage des valeurs calculées de déplacement que l'appareil **1** assure l'affichage de l'image du point focal **IF2**, en relation de l'image virtuelle de la cible **IC** de sorte que l'opérateur est à même de déplacer la cible **C** (ou le point focal **F2)** à l'aide des moyens de déplacement, de manière à mettre en coïncidence l'image virtuelle de la cible **IC** avec l'image du point focal **IF2**, avec l'aide de l'affichage du déplacement des moyens de déplacement qui est mis à jour en continu.

Il est à noter qu'il n'est pas aisé d'afficher sur une image tridimensionnelle, l'image virtuelle de la cible **C** et l'image du point focal **F2,** de sorte que cette information soit facilement intelligible par l'opérateur.

Selon une variante préférée de réalisation, l'affichage de l'image du point focal en relation de l'image virtuelle de la cible est réalisé en créant au moins deux images virtuelles **I₂**, **I₃** réalisées dans deux plans sécants orthogonaux et faisant apparaître dans chacune d'elles, l'image virtuelle de la position **ICV** de la cible et l'image virtuelle de la position **IF2V** du point focal **F2.** Tel que cela apparaît plus précisément aux **Fig. 3C** et **3D****,** l'écran **8** affiche ainsi deux images virtuelles **I₂**, **I₃** prises respectivement, par exemple, dans les plans **OY, OX** et **OZ, OY.**

L'écran **8** permet de visualiser également en continu le déplacement de la position des images virtuelles **IF2V** et **ICV** qui est consécutif au déplacement de la table **2.** Dans l'exemple illustré, il est observé le déplacement de l'image virtuelle de la position **ICV** de la cible, qui se rapproche de l'image virtuelle de la position **IF2V** du point focal jusqu'à la position de superposition **(****Fig. 3E, Fig. 3F****).** Les images virtuelles **I₂**, **I₃** permettent ainsi de suivre la mise en correspondance ou en coïncidence de l'image virtuelle de la position **ICV** de la cible avec l'image virtuelle de la position **IF2V** du point focal **F2.**

Lorsque cette position de coïncidence est atteinte, le système de traitement **3** peut alors être piloté pour assurer le traitement de la cible **C.**

Il est à noter que sur les **Fig. 3C** à **3F****,** l'image virtuelle de la position **IF2V** du point focal est représenté sous une forme elliptique correspondant au volume focal dont l'orientation spatiale est connue grâce au deuxième marqueur **24.** La comparaison des **Fig. 3C** et **3D** ou des **Fig. 3E** et **3F** permet de constater l'angulation différente du volume focal selon les plans **OY, OX** et **OZ, OY,** cette angulation étant également fonction de l'orientation du générateur **4.**

Comme il ressort de la description qui précède, la mise en coïncidence du point focal **F2** est réalisée en utilisant non pas la position réelle de la cible **C,** mais l'image virtuelle de la position de la cible **C.** En d'autres termes, si la position virtuelle de la cible **C** correspond à la position réelle de la cible **C,** alors la cible **C** coïncide avec le point focal **F2.** Toutefois, la cible **C** peut avoir bougé entre le moment de l'acquisition de la position de la cible **C** et le moment de la mise en oeuvre du traitement de la cible **C.**

Aussi, lorsque l'alignement est réalisé, le procédé selon l'invention présente de manière avantageuse une étape de validation de la coïncidence entre le point focal **F2** et la cible **C.**

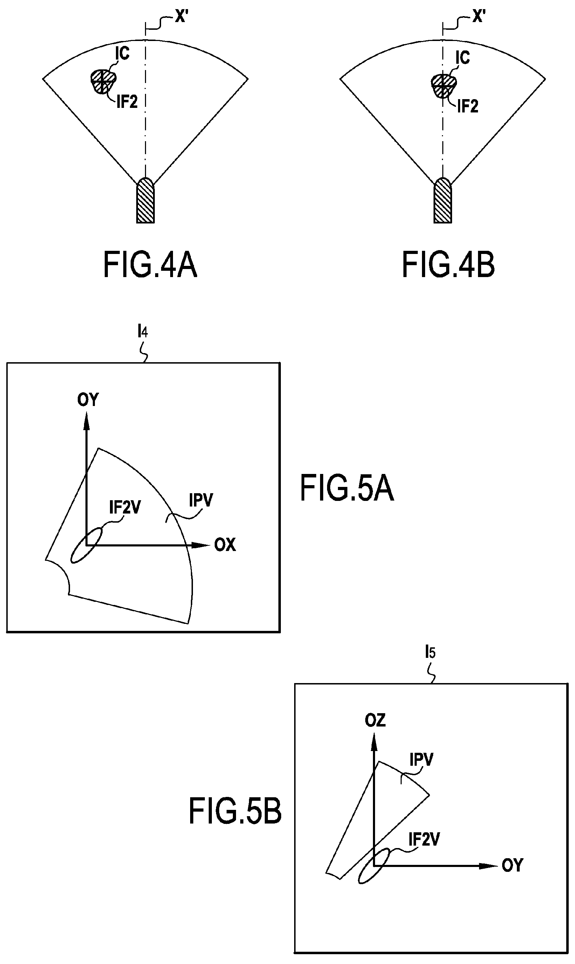

Cette étape de validation est réalisée en utilisant à nouveau la sonde ultrasonore **6** pour localiser la cible **C** et permettre d'afficher sur l'écran **8,** l'image de la cible **IC (****Fig. 4A****).** Il est à noter que cette opération de repérage de la cible **C** à l'aide de la sonde ultrasonore **6** peut être effectuée librement ou avec l'aide d'un support **35** de maintien en position de la sonde ultrasonore **6** lorsque la cible **C** est repérée. Ce support **35** qui apparaît en partie à la **Fig. 2****,** permet de maintenir la sonde ultrasonore **6** dans une position fixe offrant l'avantage de visualiser en permanence la cible **C** et ses éventuels déplacements lors du déroulement du traitement, sans contraindre l'opérateur à maintenir la sonde ultrasonore en position. Bien entendu, la sonde ultrasonore **6** peut être facilement accrochée et décrochée de ce support **35.** Par exemple, ce support **35** est formé d'un système mécanique articulé ou d'une tige souple. Ce support **35** n'est pas décrit plus précisément car il est connu en soi et ne fait pas partie de l'objet de l'invention.

Le système d'imagerie ultrasonore **5** assure l'affichage de l'image ultrasonore obtenue par la sonde ultrasonore et par suite, l'image **IC** de la position de la cible. Si la mise en coïncidence de la cible **C** avec le point focal **F2** a été correctement réalisée, alors l'image **IC** de la cible **C** est superposée à l'image **IF2** du point focal **F2.** L'opérateur peut ainsi visualiser à nouveau la cible **C** avec la sonde ultrasonore **6** selon l'incidence qui permet la meilleure qualité visuelle. Contrairement à l'état de la technique illustré à la **Fig. 4B****,** où l'axe de visée **X'** de la sonde ultrasonore **6** passe nécessairement par le point focal **F2,** l'axe de visée **X'** ne passe pas nécessairement par le point focal **F2** et ce dernier ne se trouve pas nécessairement au centre de l'image comme cela apparaît clairement à la **Fig. 4A****.** Ainsi, l'image **IF2** du point focal **F2** est affichée dans une position indépendante de l'axe de visée **X'** de la sonde ultrasonore **6.**

Dans le cas d'une coïncidence entre le point focal **F2** et la cible **C,** le système de traitement **3** est commandé pour permettre la phase de traitement de la cible **C.**

En cas de décalage entre le point focal **F2** et la cible **C,** le procédé consiste à renouveler la phase de mise en coïncidence telle que décrite ci-dessus. Ainsi, le procédé consiste à assurer de nouveau l'enregistrement de la position de la sonde ultrasonore **6** et de l'image ultrasonore **I₁** dans laquelle apparaît l'image de la cible. Cette image ultrasonore **I₁** est affichée et l'opérateur pointe la position de la cible **C.**

La position du point focal **F2** et la position de la table **2** sont enregistrées simultanément. Les valeurs de déplacement pour les moyens de déplacement sont calculées pour permettre la mise en coïncidence du point focal **F2** et de la position virtuelle de la cible **C.**

L'affichage de l'image virtuelle de la position **IF2V** du point focal en relation de l'image virtuelle de la position **ICV** de la cible est assurée. La cible **C** et/ou le point focal **F2** sont déplacés de manière qu'avec l'aide de l'affichage du déplacement des moyens de déplacement, à mettre en coïncidence l'image virtuelle de la position de la cible avec l'image virtuelle de la position du point focal. Une nouvelle étape de validation de la coïncidence du point focal **F2** et de la cible **C** est alors mise en oeuvre pour vérifier la bonne concordance entre le point focal **F2** et la cible **C.**

Tel que cela ressort de la description qui précède, l'affichage du point focal **F2** est fugitif contrairement à l'art antérieur. Par exemple, le point focal est affiché sur le plan image lorsque le plan image passe exactement sur le point focal **F2** ou à une certaine distance de ce dernier par exemple +/-5mm et de préférence +/-2mm. Pour aider l'opérateur à comprendre pourquoi le point focal disparaît de l'image ultrasonore et quelle orientation doit être donnée à la sonde ultrasonore **6** pour retrouver le point focal, l'objet de l'invention vise à faire apparaître le plan image délivré par la sonde ultrasonore **6** et le point focal **F2** dans deux images virtuelles **I₄,I₅,** comme illustré aux **Fig. 5A** et **5B**. Ces images virtuelles **I₄,I₅**, sont une projection de l'image tridimensionnelle du plan image délivré par la sonde ultrasonore **6,** sur les plans **OX,OY** et **OY,OZ.** Ainsi le plan image de la sonde ultrasonore est représenté par des projections **IPV** sur les deux plans sécants orthogonaux **OXY** et **OYZ.** Le plan image de la sonde ultrasonore **6** et ses projections **IPV** peuvent contenir l'image échographique temps réel telle que délivrée par le système d'imagerie ultrasonore **5** ou une simple représentation figurative du plan image et de ses projections. De même, le volume focal est représenté par des projections **IF2V** sur les deux plans **OXY** et **OYZ.**

Avantageusement, l'information d'altitude relative entre le plan image et le volume focal est conservée dans le calcul des projections. Cette information est retranscrite sur les projections en attribuant une semi-transparence au plan image et une opacité totale au volume focal de telle sorte que lorsque le plan image se situe en avant du volume focal, il ne masque que partiellement ce dernier et lorsqu'il se situe en arrière du volume focal, ce dernier masque en totalité le plan image.

L'invention n'est pas limitée aux exemples décrits et représentés car diverses modifications peuvent y être apportées sans sortir de son cadre.

## Revendications

1. Procédé de localisation et de visualisation d'une cible **(C)** appartenant à un mammifère, en particulier à un être humain par rapport à un point focal **(F2)** d'un système de traitement **(3)** par ondes de pression acoustiques, à l'aide d'un système d'imagerie ultrasonore **(5)** comportant une sonde ultrasonore et au moins un écran d'affichage d'images **(8),** la cible et/ou le système de traitement étant déplacés par des moyens de déplacement **(2, 30a, 30b)** pour mettre en coïncidence la cible **(C)** et le point focal **(F2), caractérisé en ce qu'**il comporte les étapes suivantes :
- réaliser une sonde ultrasonore **(6)** mobile dans l'espace indépendante mécaniquement du système de traitement **(3)** et localisée par un système de repérage à distance **(15)** lorsqu'elle se trouve située dans son volume de détection,
- déplacer la sonde ultrasonore **(6)** dans le volume de détection pour visualiser la cible **(C)** dans une image affichée sur l'écran **(8),**
- assurer l'enregistrement simultanément, d'une part d'une image ultrasonore dans laquelle apparaît l'image de la cible, et d'autre part, de la position de la sonde ultrasonore **(6),**
- sélectionner sur l'image ultrasonore enregistrée, la position de l'image de la cible afin de déterminer la position virtuelle de la cible **(C),**
- déterminer simultanément d'une part, la position du point focal **(F2)** du système de traitement **(3)** à l'aide du système de repérage à distance **(15)** et d'autre part la position des moyens de déplacement **(2-30a;30b)** de la cible et/ou du système de traitement **(3),**
- calculer, à partir de la position virtuelle de la cible **(C)** et des positions du point focal **(F2)** et des moyens de déplacement, les valeurs de déplacement pour les moyens de déplacement pour permettre une mise en coïncidence de la position virtuelle de la cible **(C)** avec le point focal (F2), **caractérisé en ce que** ledit procédé comprend les étapes consistant à:
- visualiser sur l'écran **(8),** l'image virtuelle de la cible avec l'image du point focal, et leur déplacement lors de la mise en coïncidence de l'image virtuelle de la cible avec le point focal par les moyens de déplacement,
- et déplacer la cible **(C)** et/ou le point focal **(F2)** à l'aide des moyens de déplacement et selon les valeurs de déplacement calculées, de manière à mettre en coïncidence la position virtuelle de la cible **(C)** avec le point focal **(F2).**

2. Procédé selon la revendication 1, **caractérisé en ce qu'**il consiste à déterminer la position des moyens de déplacement **(30)** à l'aide du système de positionnement à distance (**15**).

3. Procédé selon les revendications 1 et 2, **caractérisé en ce qu'**il consiste à assurer l'affichage des valeurs de déplacement calculées pour les moyens de déplacement et à mettre à jour ces valeurs de déplacement affichées en fonction du déplacement réalisé.

4. Procédé selon la revendication 1, **caractérisé en ce qu'**il consiste à assurer l'affichage de deux images virtuelles (**I₂, I₃**) réalisées dans 2 plans sécants orthogonaux et faisant apparaître dans chacune d'elles, l'image virtuelle de la position **(ICV)** de la cible et l'image virtuelle de la position **(IF2V)** du point focal, ainsi que leurs déplacements relatifs consécutifs au mouvement des moyens de déplacement.

5. Procédé selon la revendication 1, **caractérisé en ce qu'**il consiste à l'aide du système de positionnement à distance **(15),** à visualiser l'orientation du volume focal du système de traitement.

6. Procédé selon l'une des revendications 1 à 5, **caractérisé en ce qu'**il comporte une étape de validation de la coïncidence du point focal **(F2)** et de la cible **(C),** consistant :
- à déplacer la sonde ultrasonore **(6)** pour localiser la cible **(C)** pour permettre d'afficher sur l'écran, l'image de la cible **(C),**
- à assurer l'enregistrement de la position du point focal et l'affichage de l'image du point focal pour vérifier la coïncidence entre le point focal et la cible en vue de commander le système de traitement.

7. Procédé selon la revendication 5, **caractérisé en ce que** l'étape de validation consiste, en cas de décalage entre le point focal **(F2)** et la cible **(C) :**
- à déplacer la sonde ultrasonore **(6)** pour visualiser la cible **(C)** dans une image affichée sur l'écran **(8),**
- à enregistrer simultanément, d'une part l'image dans laquelle apparaît l'image de la cible et d'autre part, la position de la sonde ultrasonore **(6),**
- à sélectionner sur l'image enregistrée, la position de l'image de la cible,
- à déterminer simultanément d'une part, la position du point focal **(F2)** et d'autre part, la position des moyens de déplacement,
- à calculer les valeurs de déplacement pour les moyens de déplacement pour permettre une mise en coïncidence du point focal **(F2)** avec la position virtuelle de la cible **(C),**
et à déplacer la cible **(C)** et/ou le point focal **(F2)** à l'aide des moyens de déplacement de manière à mettre en coïncidence la position virtuelle de la cible **(C)** avec le point focal **(F2).**

8. Procédé selon la revendication 1, **caractérisé en ce qu'**il consiste à afficher l'image **(IF2)** du point focal **(F2)** dans une position indépendante de l'axe de visée **(X')** de la sonde ultrasonore **(6).**

9. Appareil de localisation et de visualisation d'une cible appartenant à un mammifère, en particulier à un être humain, cet appareil comportant :
- un système de traitement **(3)** par ondes de pression acoustiques dirigées vers un point focal,
- des moyens de déplacement **(2-30a;30b)** de la cible et/ou du système de traitement, pour mettre en coïncidence la cible **(C)** et le point focal **(F2),**
- des moyens pour déterminer la position de ces moyens de déplacement,
- un système d'imagerie ultrasonore **(5)** comportant une sonde ultrasonore **(6)** et des moyens de formation d'images **(7)** permettant de visualiser sur au moins un écran **(8),** la cible **(C)** et le point focal **(F2), caractérisé en ce qu'**il comporte :
- une sonde ultrasonore **(6)** mobile dans l'espace et indépendante mécaniquement du système de traitement **(3),**
- un système de repérage d'objets à distance **(15)** permettant de localiser le point focal **(F2)** du système de traitement **(3)** et la sonde ultrasonore **(6)** lorsque la sonde ultrasonore **(6)** et le système de traitement **(3)** sont situés dans le volume de détection dudit système de repérage,
- une unité de calcul et de commande comportant :
• des moyens pour enregistrer simultanément, d'une part, une image ultrasonore dans laquelle apparaît l'image **(IC)** de la cible **(C)** et d'autre part, la position de la sonde ultrasonore **(6)** détectée et calculée par le système de repérage à distance **(15),**
• des moyens pour sélectionner sur l'image ultrasonore enregistrée, la position de l'image de la cible afin de déterminer la position virtuelle de la cible **(C),**
• des moyens pour déterminer simultanément, d'une part, la position du point focal **(F2)** à partir du système de repérage à distance **(15)** et d'autre part, la position des moyens de déplacement,
• des moyens pour calculer, à partir de la position virtuelle de la cible **(C)** et des positions du point focal **(F2)** et des moyens de déplacement, les valeurs de déplacement pour les moyens de déplacement pour permettre une mise en coïncidence de la position virtuelle de la cible **(C)** avec le point focal (F2), l'appareil étant **caractérisé en ce qu'**il comprend également,
• des moyens pour visualiser sur l'écran **(8)** l'image virtuelle de la cible, l'image du point focal et leur déplacement lors de la mise en coïncidence de l'image virtuelle de la cible avec le point focal, par les moyens de déplacement,
• et des moyens pour commander, selon les valeurs de déplacement calculées, les moyens de déplacement pour mettre en coïncidence la position virtuelle de la cible **(C)** avec le point focal **(F2).**

10. Appareil selon la revendication 9, **caractérisé en ce que** les moyens pour déterminer la position des moyens de déplacement **(30)** font partie du système de repérage à distance **(15).**

11. Appareil selon la revendication 9 ou 10, **caractérisé en ce que** le système de repérage à distance **(15)** comporte au moins deux caméras **(17)** de préférence infrarouge comportant un volume de détection dans lequel sont placés un premier **(21),** un deuxième **(24)** et voire un troisième marqueurs **(31)** montés solidaires respectivement de la sonde ultrasonore **(6),** du système de traitement **(3),** et des moyens de déplacement de la cible **(C).**

12. Appareil selon la revendication 11, **caractérisé en ce que** le troisième marqueur **(31)** monté solidaire des moyens de déplacement est orienté selon les axes de déplacement desdits moyens de déplacement.

13. Appareil selon la revendication 10, **caractérisé en ce que** le système de repérage à distance **(15)** comporte un marqueur de calibration pour le point focal **(F2).**

14. Appareil selon l'une des revendications 9 à 13, **caractérisé en ce qu'**il comporte des moyens assurant l'affichage des valeurs de déplacement calculés pour les moyens de déplacement, ces valeurs affichées étant mises à jour en fonction du déplacement réalisé.

15. Appareil selon la revendication 9, **caractérisé en ce qu'**il comporte des moyens pour afficher deux images virtuelles **(I₂,I₃)** réalisées dans deux plans sécants et faisant apparaître dans chacune d'elles, l'image virtuelle de la position **(ICV)** de la cible et l'image virtuelle de la position **(IF2V)** du point focal, ainsi que leurs déplacements relatifs consécutifs au mouvement des moyens de déplacement.

16. Appareil selon la revendication 9, **caractérisé en ce que** les moyens d'affichage forment une image affichant le plan image délivré par la sonde ultrasonore et le point focal.

## Patentansprüche

1. Verfahren zur Lokalisation und Visualisierung eines Ziels (C), das einem Säuger, insbesondere einem Menschen, angehört, in Bezug zu einem Fokuspunkt (F2) eines Behandlungssystems (3) mit akustischen Druckwellen mit Hilfe eines Ultraschallbildgebungssystems (5), umfassend eine Ultraschallsonde und mindestens einen Bildanzeigebildschirm (8), wobei das Ziel und/oder das Behandlungssystem durch Verschiebemittel (2, 30a, 30b) verschoben werden, um das Ziel (C) und den Fokuspunkt (F2) übereinzustimmen, **dadurch gekennzeichnet, dass** es die folgenden Schritte umfasst:
- Verwirklichung einer im Raum beweglichen Ultraschallsonde (6), die mechanisch von dem Behandlungssystem (3) unabhängig ist und durch ein Fernortungssystem (15) lokalisiert wird, wenn sie sich in seinem Erfassungsvolumen befindet,
- Verschieben der Ultraschallsonde (6) in dem Erfassungsvolumen, um das Ziel (C) in einem am Bildschirm (8) angezeigten Bild zu visualisieren,
- gleichzeitige Aufzeichnung einerseits eines Ultraschallbildes, in dem das Bild des Ziels erscheint, und andererseits der Position der Ultraschallsonde (6),
- auf dem aufgezeichneten Ultraschallbild Auswahl der Position des Bildes des Ziels, um die virtuelle Position des Ziels (C) zu bestimmen,
- gleichzeitiges Bestimmen einerseits der Position des Fokuspunktes (F2) des Behandlungssystems (3) mit Hilfe des Fernortungssystems (15) und andererseits der Position der Verschiebemittel (2, 30a, 30b) des Ziels und/oder des Behandlungssystems (3),
- auf Basis der virtuellen Position des Ziels (C) und der Positionen des Fokuspunktes (F2) und der Verschiebemittel Berechnung der Verschiebewerte für die Verschiebemittel, um eine Übereinstimmung der virtuellen Position des Ziels (C) mit dem Fokuspunkt (F2) zu ermöglichen, **dadurch gekennzeichnet, dass** das Verfahren die Schritte umfasst, darin bestehend:
- auf dem Bildschirm (8) das virtuelle Bild des Ziels mit dem Bild des Fokuspunktes und ihre Verschiebung bei der Übereinstimmung des virtuellen Bildes des Ziels mit dem Fokuspunkt durch die Verschiebemittel zu visualisieren,
- und das Ziel (C) und/oder den Fokuspunkt (F2) mit Hilfe der Verschiebemittel und gemäß den berechneten Verschiebewerten zu verschieben, um die virtuelle Position des Ziels (C) mit dem Fokuspunkt (F2) übereinzustimmen.

2. Verfahren nach Anspruch 1, **dadurch gekennzeichnet, dass** es darin besteht, die Position der Verschiebemittel (30) mit Hilfe des Fernpositionierungssystems (15) zu bestimmen.

3. Verfahren nach den Ansprüchen 1 und 2, **dadurch gekennzeichnet, dass** es darin besteht, die Anzeige der berechneten Verschiebewerte für die Verschiebemittel zu gewährleisten und diese angezeigten Verschiebewerte in Abhängigkeit von der durchgeführten Verschiebung zu aktualisierten.

4. Verfahren nach Anspruch 1, **dadurch gekennzeichnet, dass** es darin besteht, die Anzeige von zwei virtuellen Bildern (I₂, I₃), die in 2 orthogonalen Schnittebenen hergestellt wurden und in jeder von ihnen das virtuelle Bild der Position (ICV) des Ziels und das virtuelle Bild der Position (IF2V) des Fokuspunktes erscheinen lassen, sowie ihrer relativen Verschiebungen nach der Bewegung der Verschiebemittel zu gewährleisten.

5. Verfahren nach Anspruch 1, **dadurch gekennzeichnet, dass** es darin besteht, mit Hilfe des Fernpositionierungssystems (15) die Ausrichtung des Fokusvolumens des Behandlungssystems zu visualisieren.

6. Verfahren nach einem der Ansprüche 1 bis 5, **dadurch gekennzeichnet, dass** es einen Schritt der Validierung der Übereinstimmung des Fokuspunktes (F2) du des Ziels (C) umfasst, darin bestehend:
- die Ultraschallsonde (6) zu verschieben, um das Ziel (C) zu lokalisieren, um die Anzeige des Bildes des Ziels (C) auf dem Bildschirm zu ermöglichen,
- die Aufzeichnung der Position des Fokuspunktes und die Anzeige des Bildes des Fokuspunktes zu gewährleisten, um die Übereinstimmung zwischen dem Fokuspunkt und dem Ziel zu überprüfen, um das Behandlungssystem zu steuern.

7. Verfahren nach Anspruch 5, **dadurch gekennzeichnet, dass** der Validierungsschritt im Falle einer Abweichung zwischen dem Fokuspunkt (F2) und dem Ziel (C) darin besteht:
- die Ultraschallsonde (6) zu verschieben, um das Ziel (C) in einem auf dem Bildschirm (8) angezeigten Bild zu visualisieren,
- gleichzeitig einerseits das Bild, in dem das Bild des Ziels erscheint, und andererseits die Position der Ultraschallsonde (6) aufzuzeichnen,
- auf dem aufgezeichneten Bild die Position des Bildes des Ziels auszuwählen,
- gleichzeitig einerseits die Position des Fokuspunktes (F2) und andererseits die Position der Verschiebemittel zu bestimmen,
- die Verschiebewerte für die Verschiebemittel zu berechnen, um eine Übereinstimmung des Fokuspunktes (F2) mit der virtuellen Position des Ziels (C) zu ermöglichen,
und das Ziel (C) und/oder den Fokuspunkt (F2) mit Hilfe der Verschiebemittel zu verschieben, um die virtuelle Position des Ziels (C) mit dem Fokuspunkt (F2) übereinzustimmen.

8. Verfahren nach Anspruch 1, **dadurch gekennzeichnet, dass** es darin besteht, das Bild (IF2) des Fokuspunktes (F2) in einer von der Sichtachse (X') der Ultraschallsonde (6) unabhängigen Position anzuzeigen.

9. Gerät zur Lokalisation und Visualisierung eines Ziels, das einem Säuger, insbesondere einem Menschen, angehört, wobei dieses Gerät umfasst:
- ein Behandlungssystem (3) mit akustischen Druckwellen, die zum Fokuspunkt gerichtet sind,
- Verschiebemittel (2, 30a, 30b) des Ziels und/oder des Behandlungssystems, um das Ziel (C) und den Fokuspunkt (F2) übereinzustimmen,
- Mittel zum Bestimmen der Position dieser Verschiebemittel,
- ein Ultraschallbildgebungssystem (5), umfassend eine Ultraschallsonde (6) und Bildformungsmittel (7), die es ermöglichen, auf mindestens einem Bildschirm (8) das Ziel (C) und den Fokuspunkt (F2) zu visualisieren,
**dadurch gekennzeichnet, dass** es umfasst:
- eine im Raum bewegliche Ultraschallsonde (6), die mechanisch von dem Behandlungssystem (3) unabhängig ist,
- ein Fernortungssystem (15) von Objekten, das es ermöglicht, den Fokuspunkt (F2) des Behandlungssystems (3) und die Ultraschallsonde (6) zu lokalisieren, wenn die Ultraschallsonde (6) und das Behandlungssystem (3) in dem Erfassungsvolumen des Ortungssystems angeordnet sind,
- eine Berechnungs- und Steuereinheit, umfassend:
* Mittel, um gleichzeitig einerseits ein Ultraschallbild, in dem das Bild (IC) des Ziels (C) erscheint, und andererseits die Position der Ultraschallsonde (6), die von dem Fernortungssystem (15) erfasst und berechnet wurde, aufzuzeichnen,
* Mittel, um auf dem aufgezeichneten Ultraschallbild die Position des Bildes des Ziels auszuwählen, um die virtuelle Position des Ziels (C) zu bestimmen,
* Mittel, um gleichzeitig einerseits die Position des Fokuspunktes (F2) auf Basis des Fernortungssystems (15) und andererseits die Position der Verschiebemittel zu bestimmen,
* Mittel, um auf Basis der virtuellen Position des Ziels (C) und der Positionen des Fokuspunktes (F2) und der Verschiebemittel die Verschiebewerte für die Verschiebemittel zu berechnen, um eine Übereinstimmung der virtuellen Position des Ziels (C) mit dem Fokuspunkt (F2) zu ermöglichen, wobei das Gerät **dadurch gekennzeichnet ist, dass** es auch umfasst:
* Mittel, um auf dem Bildschirm (8) das virtuelle Bild des Ziels, das Bild des Fokuspunktes und ihre Verschiebung bei der Übereinstimmung des virtuellen Bildes des Ziels mit dem Fokuspunkt durch die Verschiebemittel zu visualisieren,
* und Mittel, um gemäß den berechneten Verschiebewerten die Verschiebemittel zu steuern, um die virtuelle Position des Ziels (C) mit dem Fokuspunkt (F2) übereinzustimmen.

10. Gerät nach Anspruch 9, **dadurch gekennzeichnet, dass** die Mittel zur Bestimmung der Position der Verschiebemittel (30) Teil des Fernortungssystems (15) sind.

11. Gerät nach Anspruch 9 oder 10, **dadurch gekennzeichnet, dass** das Fernortungssystem (15) mindestens zwei Kameras (17), vorzugsweise Infrarotkameras, umfasst, die ein Erfassungsvolumen besitzen, in dem ein erster (21), ein zweiter (24) bzw. ein dritter Marker (31) angeordnet sind, die mit der Ultraschallsonde (6), dem Behandlungssystem (3) bzw. den Verschiebemitteln des Ziels (C) verbunden montiert sind.

12. Gerät nach Anspruch 11, **dadurch gekennzeichnet, dass** der dritte Marker (31), der mit den Verschiebemitteln verbunden montiert ist, entlang der Verschiebeachsen der Verschiebemittel ausgerichtet ist.

13. Gerät nach Anspruch 10, **dadurch gekennzeichnet, dass** das Fernortungssystem (15) einen Kalibriermarker für den Fokuspunkt (F2) umfasst.

14. Gerät nach einem der Ansprüche 9 bis 13, **dadurch gekennzeichnet, dass** es Mittel umfasst, die die Anzeige der berechneten Verschiebewerte für die Verschiebemittel gewährleisten, wobei diese angezeigten Werte in Abhängigkeit von der durchgeführten Verschiebung aktualisiert werden.

15. Gerät nach Anspruch 9, **dadurch gekennzeichnet, dass** es Mittel zur Anzeige von zwei virtuellen Bildern (I₂, I₃), die in 2 orthogonalen Schnittebenen hergestellt wurden und in jeder von ihnen das virtuelle Bild der Position (ICV) des Ziels und das virtuelle Bild der Position (IF2V) des Fokuspunktes erscheinen lassen, sowie ihrer relativen Verschiebungen nach der Bewegung der Verschiebemittel umfasst.

16. Gerät nach Anspruch 9, **dadurch gekennzeichnet, dass** die Anzeigemittel ein Bild formen, das die von der Ultraschallsonde gelieferte Bildebene und den Fokuspunkt anzeigt.

## Claims

1. A method for locating and visualizing a target **(C)** belonging to a mammal, a human being in particular, in relation to a focal point **(F2)** of a treatment system **(3)** using acoustic pressure waves, by means of an ultrasound imaging system **(5)** comprising an ultrasound probe and at least one image display screen **(8),** the target and/or the treatment system being moved by displacing means **(2, 30a, 30b)** to cause the target **(C)** to coincide with the focal point **(F2), characterized in that** it comprises the following steps:
- forming an ultrasound probe **(6)** mobile in space, mechanically independent of the treatment system **(3)** and located by a remote locating system **(15)** when it lies within the detection volume thereof,
- moving the ultrasound probe **(6)** within the detection volume to visualize the target **(C)** in an image displayed on the screen **(8),**
- ensuring the simultaneous recording firstly of an ultrasound image in which the image of the target appears, and secondly of the position of the ultrasound probe **(6),**
- selecting the position of the image of the target in the recorded ultrasound image, to determine the virtual position of the target **(C),**
- simultaneously determining firstly the position of the focal point **(F2)** of the treatment system **(3)** by means of the remote locating system **(15),** and secondly the position of the displacing means **(2-30a;30b)** moving the target and/or the treatment system **(3),**
- calculating displacement values for the displacing means, from the virtual position of the target **(C)** and from the positions of the focal point **(F2)** and of the displacing means, to allow the virtual position of the target **(C)** to coincide with the focal point **(F2), characterized in that** said method comprises the steps consisting in:
- visualizing the virtual image of the target with the image of the focal point on the screen **(8),** and the movement thereof when causing the virtual image of the target to coincide with the focal point using the displacing means,
- and moving the target **(C)** and/or focal point **(F2)** via the displacing means in accordance with the calculated displacement values, so as to cause the virtual position of the target **(C)** to coincide with the focal point **(F2).**

2. The method according to claim 1, **characterized in that** it comprises determining the position of the displacing means **(30)** by means of the remote locating system **(15).**

3. The method according to claims 1 and 2, **characterized in that** it comprises ensuring the display of the displacement values calculated for the displacing means, and updating these displacement values in relation to the movement made.

4. The method according to claim 1, **characterized in that** it comprises ensuring the display of two virtual images **(I₂**, **I₃)** taken along 2 orthogonal secant planes and causing to appear in each thereof the virtual image of the position **(ICV)** of the target and the virtual image of the position **(IF2V)** of the focal point, and their respective movements subsequent to movement of the displacing means.

5. The method according to claim 1, **characterized in that**, by means of the remote positioning device **(15),** it consists of visualizing the orientation of the focal volume of the treatment system.

6. The method according to any of claims 1 to 5, **characterized in that** it comprises a step to confirm the coinciding of the focal point **(F2)** with the target **(C),** consisting of:
- moving the ultrasound probe **(6)** to locate the target **(C)** allowing the display of the image of the target **(C)** on the screen,
- ensuring the recording of the position of the focal point and the display of the image of the focal point, to verify that the focal point and target coincide, with a view to operating the treatment system.

7. The method according to claim 5, **characterized in that** the confirmation step, in the event of offset between the focal point **(F2)** and the target(**C**) consists of:
- moving the ultrasound probe **(6)** to visualize the target **(C)** in an image displayed on the screen **(8),**
- simultaneously recording firstly the image in which the image of the target appears, and secondly the position of the ultrasound probe **(6),**
- selecting the position of the image of the target in the recorded image,
- simultaneously determining firstly the position of the focal point **(F2)** and secondly the position of the displacing means,
- calculating the displacement values for the displacing means to cause the focal point **(F2)** to coincide with the virtual position of the target **(C),**
and of moving the target **(C)** and/or focal point **(F2)** via the displacing means so as to cause the virtual position of the target **(C)** to coincide with the focal point **(F2).**

8. The method according to claim 1, **characterized in that** it consists of displaying the image **(IF2)** of the focal point **(F2)** in a position independent of the sighting axis **(X')** of the ultrasound probe **(6).**

9. An apparatus for locating and visualizing a target belonging to a mammal, a human being in particular, this apparatus comprising:
- a treatment system **(3)** with acoustic pressure waves directed towards a focal point,
- displacing means **(2-30a;30b)** to move the target and/or treatment system to cause the target **(C)** to coincide with the focal point **(F2),**
- means for determining the position of these displacing means,
- an ultrasound imaging system **(5)** comprising an ultrasound probe **(6)** and image forming means **(7)** allowing visualization of the target **(C)** and focal point **(F2)** on at least one screen **(8), characterized in that** it comprises:
- an ultrasound probe **(6)** mobile in space and mechanically independent of the treatment system **(3),**
- a system **(15)** for the remote locating of objects allowing the focal point **(F2)** of the treatment system **(3)** and the ultrasound probe **(6)** to be located when the ultrasound probe **(6)** and the treatment system **(3)** lie within the detection volume of said locating system,
- a computing and control unit comprising:
• means for simultaneously recording firstly the ultrasound image in which the image **(IC)** of the target appears **(C),** and secondly the position of the ultrasound probe **(6)** detected and calculated by the remote locating system **(15),**
• means for selecting the position of the image of the target in the recorded ultrasound image, to determine the virtual position of the target **(C),**
• means for simultaneously determining firstly the position of the focal point **(F2)** from the remote locating system **(15),** and secondly the position of the displacing means,
• means, using the virtual position of the target **(C)** and using the positions of the focal point **(F2)** and displacing means, for calculating the displacement values for the displacing means to allow the virtual position of the target **(C)** to coincide with the focal point **(F2),** the apparatus being **characterized in that** it also comprises
• means for visualizing on the screen **(8)** the virtual image of the target, the image of the focal point and their movement when the virtual image of the target is caused to coincide with the focal point using the displacing means,
• and means, in accordance with the calculated displacement values, for controlling the displacing means to cause the virtual position of the target **(C)** to coincide with the focal point **(F2).**

10. The apparatus according to claim 9, **characterized in that** the means for determining the position of the displacing means **(30)** are part of the remote locating system **(15).**

11. Apparatus according to claim 9 or 10, **characterized in that** the remote locating system **(15)** comprises at least two, preferably infrared, cameras **(17)** comprising a detection volume in which a first **(21),** a second **(24)** even a third **(31)** marker are positioned respectively attached to the ultrasound probe **(6),** the treatment system **(3),** and the means for displacing the target **(C).**

12. The apparatus according to claim 11, **characterized in that** the third marker **(31)** attached to the displacing means is oriented along the axes of movement of said displacing means.

13. The apparatus according to claim 10, **characterized in that** the remote locating system **(15)** comprises a calibration marker for the focal point **(F2).**

14. The apparatus according to any of claims 9 to 13, **characterized in that** it comprises means ensuring the display of the displacement values calculated for the displacing means, these displayed values being updated in relation to the displacement movement.

15. The apparatus according to claim 9, **characterized in that** it comprises means for displaying two virtual images **(I₂,I₃)** taken along two secant planes, and causing to appear in each thereof the virtual image of the position **(ICV)** of the target and the virtual image of the position **(IF2V)** of the focal point, and their relative movements subsequent to movement of the displacing means.

16. The apparatus according to claim 9, **characterized in that** the display means form an image displaying the image plane delivered by the ultrasound probe and the focal point.
